(19) Europäisches Patentamt
European Patent Office
Office européen des brevets

(11) **EP 4 509 859 A1**

(12) **EUROPÄISCHE PATENTANMELDUNG**

(43) Veröffentlichungstag:
**19.02.2025 Patentblatt 2025/08**

(21) Anmeldenummer: **23191605.7**

(22) Anmeldetag: **16.08.2023**

(51) Internationale Patentklassifikation (IPC):
**G01R 33/54** (2006.01)    **G01R 33/58** (2006.01)
**G01R 33/28** (2006.01)

(52) Gemeinsame Patentklassifikation (CPC):
**G01R 33/543; G01R 33/583; G16H 40/40;**
G01R 33/288

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **Siemens Healthineers AG**
**91301 Forchheim (DE)**

(72) Erfinder: **Schnetter, Volker**
**90425 Nürnberg (DE)**

(74) Vertreter: **Siemens Healthineers**
**Patent Attorneys**
**Postfach 22 16 34**
**80506 München (DE)**

(54) **ERMITTLUNG EINES PERFORMANZWERTS EINER MRT-ANLAGE**

(57)    Die Erfindung betrifft ein Verfahren zur Ermittlung eines Performanzwerts einer MRT-Anlage, eine Auswerteeinheit und ein Computerprogrammprodukt.

Gemäß dem Verfahren wird eine Referenz-Häufigkeitsverteilung von Referenz-Depositionswerten untersuchter Referenz-Objekte empfangen. Ferner wird zumindest ein Untersuchungs-Depositionswert für eine MRT-Untersuchung zumindest eines Untersuchungs-Objekts mittels der MRT-Anlage empfangen. Der Performanzwert der MRT-Anlage wird anhand der Referenz-Häufigkeitsverteilung und des zumindest einen Untersuchungs-Depositionswerts ermittelt.

FIG 3

EP 4 509 859 A1

**Beschreibung**

Die Erfindung betrifft ein Verfahren zur Ermittlung eines Performanzwerts einer MRT-Anlage, eine Auswerteeinheit und ein Computerprogrammprodukt.

In der Medizintechnik zeichnet sich die Bildgebung mittels Magnetresonanztomographie (MRT, engl. Magnetic Resonance Imaging, MRI) durch hohe Weichteilkontraste aus. Hierbei wird typischerweise ein Patient in einem Hauptmagnetfeld einer MRT-Anlage positioniert. Während einer MRT-Untersuchung werden üblicherweise mit Hilfe einer Hochfrequenzantenneneinheit einer Magnetresonanzvorrichtung hochfrequente (HF) Pulse in den Patienten eingestrahlt. Durch die erzeugten HF-Pulse werden im Patienten Kernspins angeregt, wodurch ortskodierte Magnetresonanzsignale ausgelöst werden. Die Magnetresonanzsignale werden von der MRT-Anlage empfangen und zur Rekonstruktion von Magnetresonanzabbildungen verwendet.

Für den Betreiber einer MRT-Anlage ist eine möglichst hohe Performanz wünschenswert. Die Performanz einer MRT-Anlage kann sich beispielsweise in einer Schnelligkeit der MRT-Untersuchung und/oder einer Anzahl gemessener Schichten und/oder einer resultierenden Bildqualität ausdrücken.

Aufgabe der vorliegenden Erfindung ist es, einen Performanzwerts einer MRT-Anlage zu ermitteln, um insbesondere zuverlässig mögliche Änderungen in der Performanz erkennen und bewerten zu können.

Die Aufgabe wird durch die Merkmale der unabhängigen Ansprüche gelöst. Vorteilhafte Ausgestaltungen sind in den abhängigen Ansprüchen beschrieben. Unabhängig vom grammatikalischen Geschlecht eines bestimmten Begriffes sind Personen mit männlicher, weiblicher oder anderer Geschlechteridentität mit umfasst.

Es wird ein computerimplementiertes Verfahren zur Ermittlung eines Performanzwerts einer MRT-Anlage vorgeschlagen. Dabei wird eine Referenz-Häufigkeitsverteilung von Referenz-Depositionswerten untersuchter Referenz-Objekte empfangen. Ferner wird zumindest ein Untersuchungs-Depositionswert für eine MRT-Untersuchung zumindest eines Untersuchungs-Objekts mittels der MRT-Anlage empfangen, insbesondere ermittelt. Der Performanzwert der MRT-Anlage wird anhand der Referenz-Häufigkeitsverteilung und des zumindest einen Untersuchungs-Depositionswerts ermittelt.

Die Ermittlung des Performanzwerts anhand der Referenz-Häufigkeitsverteilung erlaubt vorteilhafterweise eine statistische Auswertung der Performanz der MRT-Anlage. Somit kann die Performanz der MRT-Anlage besonders zuverlässig beurteilt werden. Ein Performanzwert einer MRT-Anlage ist vorzugsweise ein Maß für die Leistungsfähigkeit und/oder Funktionsfähigkeit der MRT-Anlage.

Das vorgeschlagene Verfahren berücksichtigt vorteilhafterweise die Patientenschwankung bei der Beurteilung der Performanz der MRT-Anlage. Systematische Abweichungen lassen sich besser von zufälligen unterscheiden.

Vorteilhafterweise handelt es sich bei den Depositionswerten um Werte, die regelmäßig ohnehin zur Durchführung von MRT-Untersuchungen ermittelt werden, so dass diese als Referenz-Depositionswerte bereits vorliegen bzw. als Untersuchungs-Depositionswerte ohnehin ermittelt werden, d.h. nicht nur zum Zwecke der Ermittlung des Performanzwerts ermittelt werden.

Die Referenz-Objekte sind üblicherweise Patienten, die mit einer oder mehreren Referenz-MRT-Anlangen untersucht wurden. Die Untersuchungs-Objekte sind üblicherweise Patienten, die mit der MRT-Anlage, für die der Performanzwert bestimmt werden soll, untersucht werden.

Die Referenz-Häufigkeitsverteilung kann insbesondere aus vorhandenen MRT-Untersuchungsdatensätzen generiert werden. Insbesondere wird dabei die Häufigkeitsverteilung der Depositionswerte der untersuchten Referenz-Objekte ermittelt. Die Häufigkeitsverteilung beschreibt dabei insbesondere eine jeweilige Anzahl der jeweiligen Referenz-Depositionswerte, welche für die in einer vorhergehenden MRT-Untersuchung untersuchten Objekte mit dem zumindest einen Objektparameterwert ermittelt wurden. Die Referenz-Depositionswerte betreffen dabei die untersuchten Referenz-Objekte, welche insbesondere einen Objektparameterwert als Untersuchungsparameterwert aufweisen. Der Objektparameterwert kann beispielsweise einen bestimmten Wert einer Masse des untersuchten Objektes oder einen bestimmten Wertebereich der Massen der untersuchten Objekte betreffen. Mit anderen Worten bezieht sich die Referenz-Häufigkeitsverteilung vorzugsweise auf die bereits untersuchten Objekte, die den zumindest einen Objektparameterwert als Untersuchungsparameterwert aufweisen. Die Häufigkeitsverteilung kann beispielsweise beschreiben, welche Depositionswerte in welcher Anzahl für untersuchte Objekte einer Masse von 80 kg in vorhergehenden MRT-Untersuchungen ermittelt wurden.

Vorzugsweise beschreiben die Depositionswerte, insbesondere die Referenz-Depositionswerte und der zumindest eine Untersuchungs-Depositionswert, jeweils einen SAR-Wert oder einen B1-Wert oder einen Leistungswert, insbesondere einen Sendeleistungswert. Ein solcher SAR-Wert kann beispielsweise ein Ganzkörper-SAR-Wert oder ein Teilbereichs-SAR-Wert sein. Ein solcher B1-Wert kann beispielsweise ein $B1+RMS$-Wert, ein $B1^2$-Wert oder ein $B1+^2$-Wert sein.

Durch Normen ist für MRT-Untersuchungen üblicherweise vorgegeben, dass die gemittelte spezifische Absorptionsrate (SAR) in einem vorgegebenen Zeitintervall einen vorgegebenen Grenzwert nicht überschreiten darf, um Schädigungen am Patienten durch Überhitzung zu vermeiden. Diese Vorgabe kann unter Umständen die Performanz (beispielsweise die Schnelligkeit der MRT-Untersuchung und/oder die Anzahl der gemessenen Schichten und/oder die resultierende Bildqualität) einschränken, da die Einhaltung der SAR-Grenzwerte es erfordern, das MRT-Protokoll der MRT-Untersuchung anzupassen, um die im Patienten abgegebene Leistung zu reduzieren.

Das MRT-Protokoll kann beispielsweise Pulsfolgen definieren, welche während der MRT-Untersuchung durch eine MRT-Anlage abzugeben sein können. Das vorgegebene MRT-Protokoll kann MRT-Protokollparameter aufweisen, welche statisch oder anpassbar sein können. Um eine Einhaltung zumindest einer Vorgabe an Depositionswerte, beispielsweise eine Einhaltung von SAR-Grenzwerten in der MRT-Untersuchung sicherstellen zu können, kann es vorgesehen sein, dass zumindest einer der MRT-Protokollparameter des vorgegebenen MRT-Protokolls in einem vorgegebenen MRT-Protokolloptimierungsverfahren angepasst oder ermittelt wird. Das MRT-Protokoll kann dazu vorgesehen sein, die MRT-Untersuchung des zu untersuchenden Objektes durch eine MRT-Anlage zu definieren. Das MRT-Protokoll kann zumindest einen MRT-Protokollparameter umfassen, wobei es sich bei dem MRT-Protokollparameter beispielsweise um einen während der MRT-Untersuchung durch die MRT-Vorrichtung zu bewirkenden Kippwinkel handeln kann.

Vorzugsweise wird der zumindest ein Untersuchungs-Depositionswert anhand eines Kalibrierungsparameters ermittelt, der unabhängig von einem vorgegebenen MRT-Protokoll der MRT-Untersuchung ist. Vorteilhafterweise ist dadurch auch der ermittelte Performanzwert unabhängig von den konkreten Einstellungen des MRT-Protokolls, wie z.B. Schichtanzahl oder Kippwinkel.

Vorzugsweise ist der Kalibrierungsparameter abhängig von der MRT-Anlage, insbesondere dessen Performanz, und/oder dem jeweiligen Untersuchungs-Objekt, insbesondere dem zu untersuchenden Körperteil des Untersuchungs-Objekts. Vorteilhafterweise wird beim Ermitteln des Performanzwerts der Einfluss des Untersuchungs-Objekts auf eine aus dem Kalibrierungsparameter abgeleitete Größe, insbesondere dem Untersuchungs-Depositionswert, mit Hilfe der Referenz-Häufigkeitsverteilung statistisch bewertet, so dass diese abgeleitete Größe eine erhöhte Aussagekraft über die MRT-Anlage, insbesondere dessen Performanz, aufweist.

Vorzugsweise beschreibt der Kalibrierungsparameter einen Anlagensteuerwert zur Erzeugung eines Kalibrierungs-Hochfrequenzpulses mittels der MRT-Anlage.

Zur Ermittlung des Kalibrierungsparameters wird beispielsweise beim sogenannten Adjustment, einer üblicherweise vor der eigentlichen MRT-Untersuchung durchgeführten Voruntersuchung, ein Hochfrequenzpuls in ein Sendesystem der MRT-Anlage eingespeist. Die Amplitude des Hochfrequenzpulses wird so angepasst (üblicherweise: erhöht), bis eine Kalibrierungsfeldstärke B1+, die einem definierten Flip-Winkel entspricht, durch die MRT-Anlage erzeugt werden kann. Der mit der angepassten Amplitude erzeugte Hochfrequenzpuls ist dann der Kalibrierungs-Hochfrequenzpuls. Mit der angepassten Amplitude als Anlagensteuerwert werden die auszuspielenden Hochfrequenzpulse eines MRT-Protokolls, welches einen zeitlichen Ablauf der Ansteuerung definiert, skaliert. Daraus kann die vom Sendesystem abzugebende mittlere Leistung vorherbestimmt werden. Von dieser Sendeleistung wird vorzugsweise die im Sendesystem, insbesondere in einer Sendespule, verbleibende Spulenverlustleistung abgezogen, um die im Patienten absorbierte Wirkleistung zu bestimmen. Teilt man diese Wirkleistung durch die Masse des Untersuchungs-Objekts, erhält man den SAR-Wert, insbesondere einen Ganzkörper-SAR-Wert.

Ferner ist es auch denkbar, eine definierte Leistung einzustellen und den daraus resultierenden B1+-Wert zu messen.

Neben der Masse des Untersuchungs-Objekts kann die Leistung - und somit die SAR, die für den Kalibrierungs-Hochfrequenzpulses benötigt wird - von weiteren Untersuchungsparametern abhängen, wie beispielsweise von der Konstitution des Untersuchungs-Objekts, von einer Lage eines Untersuchungsbereichs des Untersuchungs-Objekts und/oder eine Anregungsform zur Anregung des Untersuchungs-Objekts. Diese Untersuchungsparameter sind vorzugsweise nicht abhängig von einem spezifischen MRT-Protokoll.

Vorzugsweise umfasst das Verfahren ferner ein Empfangen zumindest eines Untersuchungsparameterwerts der MRT-Untersuchung, wobei die Referenz-Häufigkeitsverteilung aus vorliegenden MRT-Untersuchungsdatensätzen von untersuchten Referenz-Objekten ermittelt ist, die jeweils den zumindest einen Untersuchungsparameterwert aufweisen und/oder deren zumindest eine Untersuchungsparameterwert in einem Bereich liegt, der dem empfangenen zumindest einen Untersuchungsparameterwert zugeordnet ist.

Der zumindest eine Untersuchungsparameterwert der MRT-Untersuchung kann beispielsweise durch ein Bedienpersonal eingegeben und/oder automatisch gemäß dem verwendeten MRT-Protokoll ermittelt werden und durch eine Empfangseinheit einer Auswerteeinheit zur Ermittlung des Performanzwerts empfangen werden.

Vorzugsweise umfasst ein jeweiliger der MRT-Untersuchungsdatensätze den jeweiligen Untersuchungs-Depositionswert und den jeweiligen Untersuchungsparameterwert der jeweiligen MRT-Untersuchung.

Vorzugsweise ist der der zumindest eine Untersuchungsparameterwert dadurch gekennzeichnet, dass er eine Deposition des Untersuchungs-Objektes in der MRT-Untersuchung beeinflussen kann. Vorzugsweise umfasst der zumindest eine Untersuchungsparameterwert eine Masse des zumindest einen Untersuchungs-Objekts und/oder eine Lage eines Untersuchungsbereichs des Untersuchungs-Objekts und/oder eine Anregungsform zur Anregung des Untersuchungs-Objekts und/oder eine Zugehörigkeit des Untersuchungs-Objekts zu einer Personengruppe umfasst. Die Lage eines Untersuchungsbereichs des Untersuchungs-Objekts kann beispielsweise durch das zu untersuchende Körperteil des Untersuchungs-Objekts und/oder durch die Untersuchungsposition des Untersuchungs-Objekts gekennzeichnet sein.

Die Anregungsform zur Anregung des Untersuchungs-Objekts kann beispielsweise dadurch charakterisiert sein, ob es sich um eine zirkular polarisierte (engl. circularly polarized, CP) oder um eine elliptisch polarisierte (elliptically polarized, EP) Anregung handelt.

Die Personengruppe kann beispielsweise durch das Geschlecht des zumindest einen Untersuchungs-Objekts charakterisiert sein. Eine andere sinnvolle Unterscheidung wäre beispielsweise Leistungssportler versus Nichtleistungssportler, da Leistungssportler aufgrund ihres höheren Muskelanteils zur Erzeugung eines bestimmten Magnetfeldes eine höhere Sendeleistung benötigen.

Vorzugsweise ist die Referenz-Häufigkeitsverteilung aus vorliegenden MRT-Untersuchungsdatensätzen mehrerer MRT-Referenz-Anlagen ermittelt. Vorteilhafterweise wird dadurch die Datenbasis zur Ermittlung der Referenz-Häufigkeitsverteilung vergrößert und/oder die Genauigkeit der Referenz-Häufigkeitsverteilung erhöht. Dabei sind die mehreren MRT-Referenz-Anlagen vorzugsweise gleichen Typs, um die Aussagekraft aus der Referenz-Häufigkeitsverteilung ermittelten Performanzwerts zu erhöhen.

Vorzugsweise umfasst das Ermitteln des Performanzwerts einen Vergleich des zumindest einen Untersuchungs-Depositionswerts mit der Referenz-Häufigkeitsverteilung und/oder eine Einordnung des zumindest einen Untersuchungs-Depositionswerts in die Referenz-Häufigkeitsverteilung.

Vorzugsweise wird die Referenz-Häufigkeitsverteilung in Form einer, insbesondere relativen, Summenhäufigkeit (auch kumulierte Häufigkeit genannt) oder einer Verteilungsfunktion empfangen und/oder aus einer Häufigkeit abgeleitet.

Die (relative) Summenhäufigkeit gibt vorzugsweise eine kumulierte Prozentzahl der Referenz-Depositionswerte bis zu dem Untersuchungs-Depositionswert an. Vorzugsweise sind zur Ableitung der Summenhäufigkeit aus der Häufigkeit die Anzahlen der Referenz-Depositionswerte gemäß der Häufigkeit nach ihrer Reihenfolge zu kumulieren, insbesondere aufzuaddieren. Die Verteilungsfunktion gibt vorzugsweise an, mit welcher Wahrscheinlichkeit ein Referenz-Depositionswert höchstens den Untersuchungs-Depositionswert annimmt.

Vorzugsweise umfasst das Ermitteln des Performanzwerts ein Ermitteln zumindest eines Wahrscheinlichkeitswerts, der die Wahrscheinlichkeit beschreibt, dass ein Referenz-Objekt gemäß der Referenz-Häufigkeitsverteilung einen geringeren Depositionswert aufweist als der (mit der MRT-Anlage ermittelte) zumindest eine Untersuchungs-Depositionswert, oder der die Wahrscheinlichkeit beschreibt, dass ein Referenz-Objekt gemäß der Referenz-Häufigkeitsverteilung einen höheren Untersuchungs-Depositionswert aufweist als der (mit der MRT-Anlage ermittelte) zumindest eine Untersuchungs-Depositionswert.

Vorteilhafterweise kann anhand der Referenz-Verteilungsfunktion bei jeder MRT-Untersuchung mit der Kenntnis des (aktuellen) Untersuchungs-Depositionswertes die Wahrscheinlichkeit ermitteln werden, wieviel Prozent der (Durchschnitts-)Bevölkerung (mit dem gleichen zumindest einen Untersuchungsparameterwerts, wie z.B. Gewicht und/oder Untersuchungsposition) einen kleineren Depositionswertes haben; vorteilhafterweise kann aus einem kleineren Prozentwert ein besserer Performanzwert abgeleitet werden.

Vorzugsweise umfasst der zumindest eine Untersuchungs-Depositionswert mehrere Untersuchungs-Depositionswerte, wobei das Ermitteln des Performanzwerts ein Ermitteln jeweils eines Wahrscheinlichkeitswerts für jeden der mehreren Untersuchungs-Depositionswerte und ein Ermitteln eines Mittelwerts oder Medianwerts aus den mehreren Wahrscheinlichkeitswerten. Der Mittelwert kann beispielsweise ein arithmetischer oder geometrischer Mittelwert sein.

Der Medianwert ist vorzugsweise der Wert, der genau in der Mitte der mehreren Wahrscheinlichkeitswerte liegt, die nach ihrer Größe geordnet sind. Aufgrund dieser zentralen Lage wird der Medianwert auch oftmals auch Zentralwert genannt. Der Medianwert halbiert die Wahrscheinlichkeitswerte, so dass eine Hälfte der Wahrscheinlichkeitswerte unterhalb und die andere Hälfte oberhalb des Medianwertes in der geordneten Reihe liegt.

Vorzugsweise werden die mehreren Wahrscheinlichkeitswerte zum Ermitteln des Mittelwerts oder Medianwerts anhand von Untersuchungs-Depositionswerten ermittelt, die in einem ausgewählten Zeitintervall ermittelt wurden. Beispielsweise werden die Wahrscheinlichkeitswerte über einen Zeitintervall von einer Woche oder einen Monat gemittelt. Dadurch kann vorteilhafterweise der Einfluss von Ausreißern in den Wahrscheinlichkeitswerten, der den Performanzwert möglicherweise verfälschen könnte, besser unterdrückt werden.

Ferner wird eine Auswerteeinheit vorgeschlagen, die ausgebildet ist, ein vorangehend beschriebenes Verfahren zur Ermittlung eines Performanzwerts einer MRT-Anlage auszuführen.

Die Auswerteeinheit umfasst beispielsweise eine Empfangseinheit zum Empfangen einer Referenz-Häufigkeitsverteilung von Referenz-Depositionswerten untersuchter Referenz-Objekte, und eine Empfangseinheit zum Empfangen zumindest eines Untersuchungs-Depositionswerts für eine MRT-Untersuchung zumindest eines Untersuchungs-Objekts mittels der MRT-Anlage und eine Ermittlungseinheit zum Ermitteln des Performanzwerts der MRT-Anlage anhand der Referenz-Häufigkeitsverteilung und des zumindest einen Untersuchungs-Depositionswerts. Insbesondere kann die Auswerteeinheit einen oder mehrere Prozessoren und/oder Speichermodule umfassen, um beispielsweise Rechenoperationen zur Durchführung des Verfahrens ausführen zu können. Ferner kann die Auswerteeinheit beispielsweise eine Benutzerschnittstelle mit einer Anzeigeeinheit, beispielsweise auf zumindest einem Monitor, und/oder eine Eingabeeinheit umfassen, um beispielsweise ermittelte Performanzwerte darstellen und/oder auswerten zu können.

Die Auswerteeinheit kann beispielsweise Bestandteil der MRT-Anlage sein, dessen Performanz ermittelt wird. Insbesondere kann sie Bestandteil einer Systemsteuereinheit der MRT-Anlage sein. Sie kann aber auch separat von der MRT-Anlage ausgebildet sein. Sie kann insbesondere über ein Datennetzwerk mit der MRT-Anlage verbunden sein. Das Datennetzwerk kann beispielsweise ein lokales Netzwerk. Die Auswerteeinheit kann auch über das Internet mit der MRT-Anlage verbunden sein. Die Auswerteeinheit kann insbesondere an einem anderen Ort, insbesondere einer anderen Region und/oder einem anderen Land, als die MRT-Anlage befindlich sein. Vorteilhafterweise kann die MRT-Anlage bzw. ihre Performanz aus der Ferne überwachbar sein.

Ferner wird ein Computerprogrammprodukt vorgeschlagen, das ein Programm umfasst und direkt in einen Speicher einer programmierbaren Auswerteeinheit ladbar ist und Programmmittel, z.B. Bibliotheken und Hilfsfunktionen, aufweist, um ein vorgeschlagenes Verfahren auszuführen, wenn das Computerprogrammprodukt in der Auswerteeinheit ausgeführt wird. Das Computerprogrammprodukt kann dabei eine Software mit einen Quellcode, der noch kompiliert und gebunden oder der nur interpretiert werden muss, oder einen ausführbaren Softwarecode umfassen, der zur Ausführung nur noch in die Auswerteeinheit zu laden ist.

Durch das Computerprogrammprodukt kann das vorgeschlagene Verfahren vorteilhafterweise schnell, identisch wiederholbar und robust ausgeführt werden. Das Computerprogrammprodukt ist vorzugsweise so konfiguriert, dass es mittels der Auswerteeinheit die vorgeschlagenen Verfahrensschritte ausführen kann. Die Systemsteuereinheit weist dabei jeweils die Voraus-setzungen wie beispielsweise einen entsprechenden Arbeits-speicher, eine entsprechende Grafikkarte oder eine entsprechende Logikeinheit auf, so dass die jeweiligen Verfahrens-schritte effizient ausgeführt werden können.

Das Computerprogrammprodukt ist beispielsweise auf einem computerlesbaren Medium gespeichert oder auf einem Netzwerk oder Server hinterlegt, von wo es in den Prozessor einer lokalen Auswerteeinheit geladen werden kann, der mit MRT-Anlage direkt verbunden oder als Teil der MRT-Anlage ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in der Auswerteeinheit ein vorgeschlagenes Verfahren durchführen.

Beispiele für elektronische lesbare Datenträger sind eine DVD, ein Magnetband oder einen USB-Stick, auf welchem elektronisch lesbare Steuerinformationen, insbesondere Software, gespeichert ist. Wenn diese Steuerinformationen von dem Datenträger gelesen und in eine Auswerteeinheit gespeichert werden, können alle vorgeschlagenen Ausführungsformen der vorab beschriebenen Verfahren durchgeführt werden.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:

Fig. 1    eine Auswerteeinheit mit einer MRT-Anlage sowie mehreren weiteren MRT-Anlagen,

Fig. 2    ein Verfahren zur Ermittlung eines Performanzwerts einer MRT-Anlage,

Fig. 3    eine Referenz-Häufigkeitsverteilung von Referenz-Depositionswerten zum Ermitteln eines Wahrscheinlichkeitswerts,

Fig. 4    ein zeitlicher Verlauf von Performanzwerten.

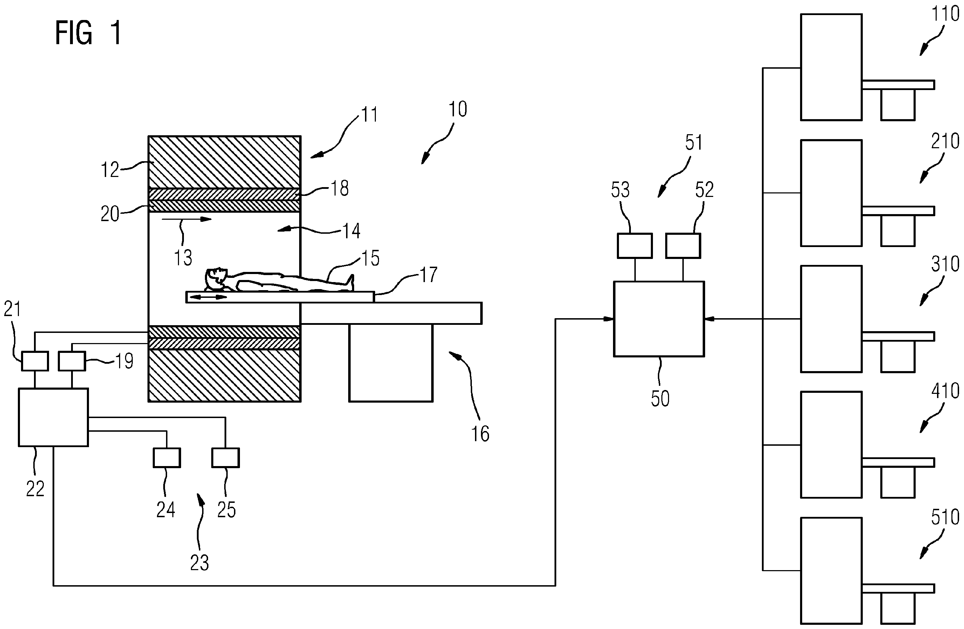

In Fig. 1 ist eine MRT-Anlage 10 schematisch dargestellt. Die MRT-Anlage 10 umfasst eine Magneteinheit 11, die einen Hauptmagneten 12 zu einem Erzeugen eines starken und insbesondere zeitlich konstanten Hauptmagnetfelds 13 aufweist. Zudem umfasst die MRT-Anlage 10 einen Patientenaufnahmebereich 14 zu einer Aufnahme eines Patienten 15 als Untersuchungs-Objekt. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der MRT-Anlage 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf. Das Sichtfeld der MRT-Anlage liegt üblicherweise in der Mitte des Patientenaufnahmebereichs 14. Somit ändert sich eine Lage eines Untersuchungsbereichs des Patienten 15, je nachdem an welcher Stelle der Patiententisch 17 mit dem Patienten 15 bewegt wird.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der MRT-Anlage 10 gesteuert. Die Magneteinheit 11 umfasst weiterhin eine Hochfrequenzantenneneinheit 20, welche im vorliegenden Ausführungsbeispiel als fest in die MRT-Anlage 10 integrierte Körperspule ausgebildet ist. Die Hochfrequenzantenneneinheit 20 wird von einer Hochfrequenzantennensteuereinheit 21 der MRT-Anlage 10 gesteuert und strahlt Hochfrequenzpulse in einen Untersuchungsraum ein, der im Wesentlichen von einem Patientenaufnahmebereich 14 der Magnetresonanzvorrichtung 10 gebildet ist, d.h. die Hochfrequenzantenneneinheit 20 und die Hochfrequenzantennensteuereinheit 21 bilden hier sein Sendesystem der MRT-Anlage. Durch das Einstrahlen der Hochfrequenzpulse stellt sich dem von dem Hauptmagneten 12 erzeugten Hauptmagnetfeld 13 eine Anregung von Atomkernen ein. Durch Relaxation der angeregten Atomkerne werden Magnetresonanzsignale erzeugt. Die Hochfrequenzantenneneinheit 20 ist zum Empfang der Magnetresonanzsignale ausgebildet.

Zu einer Steuerung des Hauptmagneten 12, der Gradientensteuereinheit 19 und zur Steuerung der Hochfrequenzantennensteuereinheit 21 weist die MRT-Anlage 10 eine Systemsteuereinheit 22 auf. Die Systemsteuereinheit 22 steuert zentral die MRT-Anlage 10, wie beispielsweise das Durchführen einer vorbestimmten MRT-Protokolls. Zudem umfasst die Systemsteuereinheit 22 eine nicht näher dargestellte Rekonstruktionseinheit zu einer Rekonstruktion der Magnetresonanzsignale, die während der Magnetresonanzuntersuchung erfasst werden. Des Weiteren umfasst die MRT-Anlage 10 eine Benutzerschnittstelle 23, die mit der Systemsteuereinheit 22 verbunden ist. Steuerinformationen wie beispielsweise Bildgebungsparameter, sowie rekonstruierte Magnetresonanzabbildungen können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

Die Systemsteuereinheit 22 der MRT-Anlage 10 ist mit einer Auswerteeinheit 50 verbunden. Die Systemsteuereinheit 22 übermittelt an die Auswerteeinheit 50 zumindest einen Untersuchungs-Depositionswert und/oder Informationen zur Ermittlung zumindest eines Untersuchungs-Depositionswerts.

Ferner ist die Auswerteeinheit 50 mit mehreren weiteren MRT-Anlagen 110, 210, 310, 410, 510 verbunden. Diese weiteren MRT-Anlagen 110, 210, 310, 410, 510 übermitteln an die Auswerteeinheit 50 Informationen zur Ermittlung einer Referenz-Häufigkeitsverteilung von Referenz-Depositionswerten von Patienten, die mit diesen weiteren MRT-Anlagen 110, 210, 310, 410, 510 untersucht wurden. Die weiteren Anlagen 110, 210, 310, 410, 510 können daher auch als Referenz-MRT-Anlagen angesehen werden.

Des Weiteren umfasst die Auswerteeinheit 50 eine Benutzerschnittstelle 51 mit einer Anzeigeeinheit 52, beispielsweise ein Monitor, und einer Eingabeeinheit 53 auf, mittels der Informationen, beispielsweise Performanzwerte der MRT-Anlage 10, einem Bedienpersonal angezeigt und/oder Informationen durch ein Bedienpersonal eingegeben werden können.

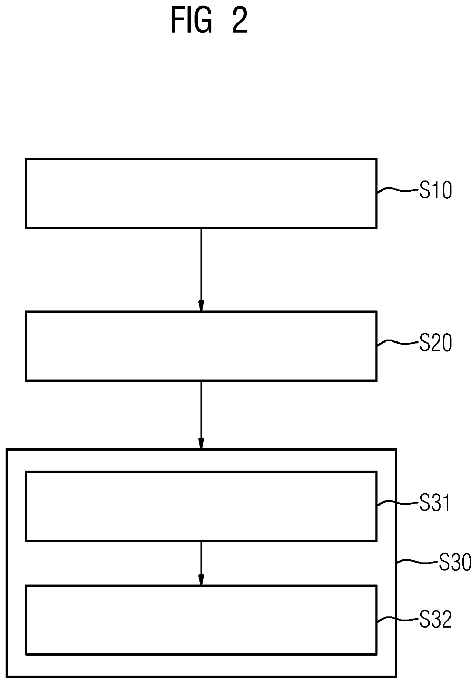

Die Auswerteeinheit 50 umfasst zumindest einen Prozessor und/oder zumindest ein Speichermodul, um ein computerimplementiertes Verfahren zur Ermittlung eines Performanzwerts einer MRT-Anlage 10 ausführen zu können. Ein solches Verfahren ist in Fig. 2 schematisch dargestellt.

In S10 wird eine Referenz-Häufigkeitsverteilung von Referenz-Depositionswerten untersuchter Referenz-Objekte empfangen. Die Referenz-Objekte sind insbesondere Patienten, die mit Referenz-MRT-Anlagen, beispielsweise den mehreren weiteren MRT-Anlagen 110, 210, 310, 410, 510, untersucht wurden. Es ist denkbar, dass es sich bei den mehreren weiteren MRT-Anlagen nicht nur um fünf MRT-Anlagen (wie dargestellt), sondern um eine ganze Flotte an MRT-Anlagen handelt.

In S20 wird zumindest ein Untersuchungs-Depositionswert für eine MRT-Untersuchung zumindest eines Untersuchungs-Objekts, insbesondere des Patienten 15, mittels der MRT-Anlage 10 ermittelt und/oder empfangen.

In S30 wird ein Performanzwert der MRT-Anlage anhand der Referenz-Häufigkeitsverteilung und des zumindest einen Depositionswerts ermittelt. Insbesondere können zur Ermittlung des Performanzwerts in S31 mehrere Wahrscheinlichkeitswerte ermittelt werden, die in S32 gemittelt werden.

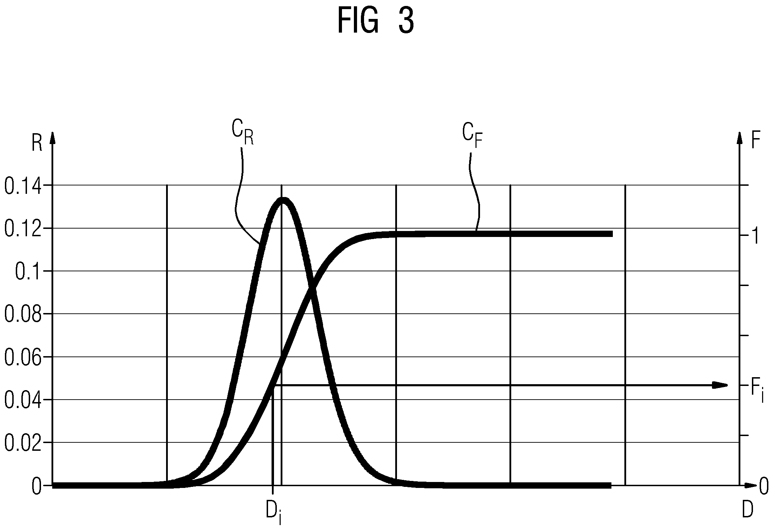

Anhand von Fig. 3 soll das Verfahren näher erläutert werden. Hier ist eine Referenz-Häufigkeitsverteilung von Referenz-Depositionswerten untersuchter Referenz-Objekte in Form einer relativen Häufigkeit $C_R$ und einer relativen Summenhäufigkeit $C_F$ dargestellt. Die relative Häufigkeit $C_R$ lässt sich üblicherweise aus einer absoluten Häufigkeit ableiten. Die linke vertikale Achse R zeigt den Wert R der relativen Häufigkeit $C_R$ an. die rechte vertikale Achse R zeigt den Wert F der relativen Summenhäufigkeit $C_F$, der zwischen 0 und 1 bzw. 100 Prozent liegt. Meist weist die Häufigkeit die Form einer Gauß-Verteilung auf, wie im dargestellten Fall.

Die horizontale Achse D zeigt einen Depositionswert an. Der Depositionswert kann beispielsweise ein SAR-Wert sein, aber auch andere Arten von Depositionswerten sind denkbar. Der SAR-Wert der sich beispielsweise wie folgt berechnen lässt: $D = P / m$

Hierbei ist P die vom Patienten absorbierte Leistung, die zur Erzeugung eines Kalibrierungs-Hochfrequenzpulses mittels des Sendesystems der MRT-Anlage 10 nötig ist. Die mittels einer solchen Kalibrierung ermittelte absorbierte Leistung P kann also als Kalibrierungsparameter angesehen werden.

Da es sich bei dem Kalibrierungs-Hochfrequenzpuls vorzugsweise um einen Hochfrequenzpuls handelt, der unabhängig von einem bei der MRT-Untersuchung auszuführenden MRT-Protokoll ist, ist auch die resultierende absorbierte Leistung im Patienten davon unabhängig. Dieser Wert kann beispielsweise in einer vor der eigentlichen MRT-Untersuchung durchzuführenden Voruntersuchung bestimmt werden.

Bei m handelt es sich um die Masse des Patienten 15, d.h. mittels dieser Größe wird D auf die Masse des Patienten 15 normiert.

Üblicherweise wird der SAR-Wert aus Sicherheitsgründen ohnehin zur Durchführung einer MRT-Untersuchung ermittelt, um etwaige SAR-Limitierungen nicht zu überschreiten. Somit steht der Depositionswert D in der Regel ohne weiteren Aufwand zur Verfügung, weshalb sich der Performanzwert besonders effizient bestimmen lässt.

Wird nun als SAR-Wert beispielsweise der Untersuchungs-Depositionswert $D_i$ ermittelt, lässt sich aus der relativen Summenhäufigkeit $C_F$ der Wahrscheinlichkeitswert $F_i = C_F(D_i)$ bestimmen. Dieser Wahrscheinlichkeitswert $F_i$ beschreibt die Wahrscheinlichkeit, dass ein Referenz-Objekt gemäß der Referenz-Häufigkeitsverteilung einen geringeren SAR-Wert aufweist als der Untersuchungs-Depositionswert $D_i$. Folglich beschreibt 1-$F_i$ die Wahrscheinlichkeit, dass ein Referenz-Objekt gemäß der Referenz-Häufigkeitsverteilung einen höheren SAR-Wert aufweist als der Untersuchungs-Depositionswert $D_i$. Ein Wahrscheinlichkeitswert von 0,5 würde bedeuten, dass das MRT-System 10 gegenüber den Referent-MRT-Systemen ein durchschnittliches Verhalten, insbesondere Performanz, zeigt. Im dargestellten Beispiel beträgt der Wahrscheinlichkeitswert $F_i$ etwa 0,4.

Vorzugsweise können verschiedene untersuchungsparameterspezifische Referenz-Häufigkeitsverteilung bereitgestellt werden. Aus den Referenz-Häufigkeitsverteilung wird gemäß zumindest einem Untersuchungsparameterwert der MRT-Untersuchung mittels der MRT-Anlage 10 eine passende Referenz-Häufigkeitsverteilung ausgewählt bzw. durch die Auswerteeinheit 50 empfangen. Geeignete Untersuchungsparameterwerte können die Masse Patienten 15 und/oder eine Lage eines Untersuchungsbereichs des Patienten 15 und/oder eine Anregungsform zur Anregung des Patienten 15 und/oder eine Zugehörigkeit des Patienten 15 zu einer Personengruppe. Beispielsweise kann die Referenz-Häufigkeitsverteilung spezifisch sein für 60-65 kg schwere Frauen, deren Köpfe mit einer CP-Anregung untersucht werden. Die Lage eines Untersuchungsbereichs des Patienten 15 kann auch in Form einer Position des Patiententisches 17 entlang seines Verfahrweges definiert sein, die üblicherweise mit einem Körperteil des Patienten 15 korrespondiert.

Die zum Ermitteln des Performanzwerts verwendete Referenz-Häufigkeitsverteilung ist vorzugsweise aus vorliegenden MRT-Untersuchungsdatensätzen von untersuchten Referenz-Objekten ermittelt, die jeweils den zumindest einen Untersuchungsparameterwert aufweisen und/oder deren zumindest einer Untersuchungsparameterwert in einem Bereich liegt, der dem empfangenen zumindest einen Untersuchungsparameterwert zugeordnet ist. Beispielsweise kann ein entsprechender Massenbereich eine Breite von 5 kg aufweisen oder ein Bereich einer Position des Patiententisches eine Breite von 10-15%.

Die MRT-Untersuchungsdatensätze können beispielsweise von den weiteren MRT-Anlagen 110, 210, 310, 410, 510 erzeugt werden. Idealerweise stützt sich die Referenz-Häufigkeitsverteilung auf möglichst viele zuvor durchgeführte MRT-Untersuchungen. Die weiteren MRT-Anlagen 110, 210, 310, 410, 510 sind vorzugsweise von demselben Typ wie die MRT-Anlage 10.

Vorzugsweise sind alle verfügbaren Referenz-Häufigkeitsverteilungen in einer zentralen Datenbank abgelegt, aus der eine jeweils passende Referenz-Häufigkeitsverteilung zur Ermittlung des Performanzwerts abgerufen werden kann und insbesondere durch die Auswerteeinheit 50 empfangen werden kann. Die Datenbank kann auch Bestandteil der Auswerteeinheit 50 sein.

Vorzugsweise stellen mehrere, idealerweise alle, MRT-Anlagen, (beispielgemäß also auch die MRT-Anlage 10 neben den weiteren MRT-Anlagen 110, 210, 310, 410, 510) Informationen, insbesondere ermittelte Depositionswerte, zur Erzeugung der Referenz-Häufigkeitsverteilungen bereit. Solche bereitstellenden MRT-Anlagen können in Sinne der Erfindung auch als MRT-Referenz-Anlagen bezeichnet werden.

Es ist möglich, dass allein aus einem einzelnen ermittelten Wahrscheinlichkeitswert $F_i$ ein Performanzwert ermittelt wird. Im einfachsten Fall ist der Performanzwert identisch mit dem ermittelten Wahrscheinlichkeitswert $F_i$, d.h. P = $F_i$.

Es ist aber auch möglich, dass mehrere MRT-Untersuchungen an der MRT-Anlage 10 durchgeführt werden, bei der jeweils ein Untersuchungs-Depositionswert, insbesondere ein SAR-Wert, ermittelt wird. Somit liegen mehrere Untersuchungs-Depositionswerte vor. Für jeden der mehrere Untersuchungs-Depositionswerte kann dann jeweils ein Wahrscheinlichkeitswert $F_x$ ermittelt werden. Aus diesen Wahrscheinlichkeitswerten $F_x$ kann insbesondere ein Mittelwert oder Medianwert ermittelt werden.

Beispielsweise kann ein arithmetischer Mittelwert gebildet werden gemäß:

$$\overline{F}_{arithm} = \frac{1}{n} \sum_{x=1}^{n} F_x$$

Oder es beispielsweise kann ein geometrischer Mittelwert gebildet werden gemäß:

$$\overline{F}_{geom} = \sqrt[n]{\prod_{x=1}^{n} F_x}$$

Insbesondere kann die Mittelung über Wahrscheinlichkeitswerten $F_x$ durchgeführt werden, die aus MRT-Untersuchungen abgeleitet werden, die in einem gewissen Zeitintervall stattfanden. Beispielsweise kann wochen- oder monatsweise ein Performanzwert ermittelt werden.

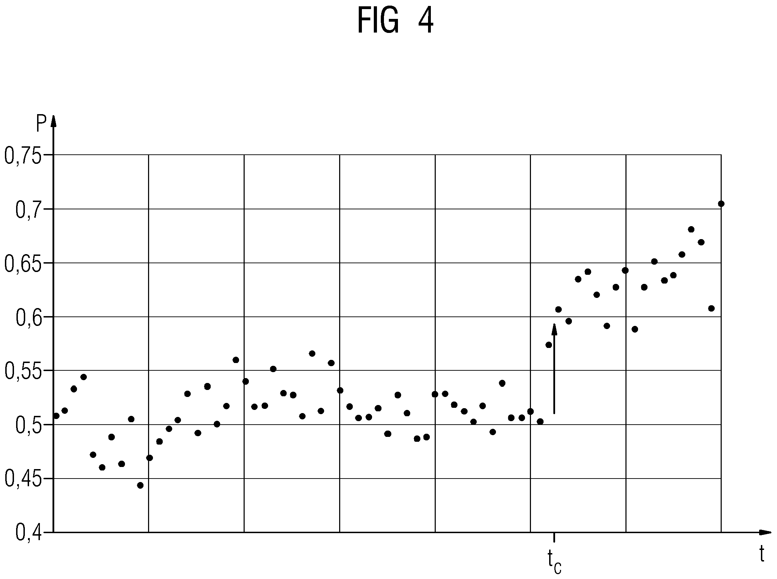

In Fig. 4 ist ein möglicher zeitlicher Verlauf des Performanzwerts einer MRT-Anlage 10 dargestellt. Jeder Punkt entspricht dabei beispielhaft einem Performanzwert $P_x$ zu einer bestimmten Zeit t. Aus der Darstellung lässt sich entnehmen, dass ab dem Zeitpunkt $t_c$ der Performanzwert P deutlich angestiegen ist. Dies deutet darauf hin, dass eine Änderung an der MRT-Anlage 10 stattgefunden haben kann. Diese Änderung kann beispielsweise durch eine Verschlechterung einer Komponente der MRT-Ablage 10 hervorgerufen worden sein. Insbesondere kann gemäß dem dargestellten Beispiel ein höherer Performanzwert einer höheren Amplitude entsprechen, die notwendig ist, um einen Kalibrierungs-Hochfrequenzpuls durch die MRT-Anlage 10 zu erzeugen. Durch die Ermittlung des Performanzwerts, bei der statistische Streuungen mit Hilfe der Referenz-Häufigkeitsverteilung berücksichtigt werden, können vorteilhafterweise mögliche Probleme frühzeitig und zuverlässig erkannt und proaktiv beseitigt werden, wie z.B. defekte oder gealterte Sendeelemente. Etwaige Anlagenausfälle können so möglicherweise verhindert werden. Aber auch der Einfluss von etwaigen Softwareupdates kann mit Hilfe der ermittelten Performanzwerte beobachtet werden.

Vorteilhafterweise können mit Hilfe des vorgeschlagenen Verfahrens zur Ermittlung der Performanzwerte unterschiedliche mögliche Einflüsse für ein bestimmtes Verhalten einer MRT-Anlage voneinander unterschieden werden, wie z.B. der Einfluss eines verwendeten MRT-Protokolls, eines untersuchten Patienten oder der MRT-Anlage selbst. Der Einfluss des individuellen Patienten kann vorteilhafterweise mit Hilfe des Performanzwerts reduziert, idealerweise eliminiert, werden, indem eine statistische Betrachtung über mehrere Patienten vorgenommen wird. Schließlich ist es wahrscheinlich, dass die durchschnittlichen Eigenschaften der Patienten gleich sind und nicht abhängig von der konkreten MRT-Anlage, mit der sie untersucht werden.

Es wird abschließend noch einmal darauf hingewiesen, dass es sich bei den vorhergehend detailliert beschriebenen Verfahren sowie bei der dargestellten Auswerteeinheit und MRT-Anlage lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass die betreffenden Komponenten aus mehreren zusammenwirkenden Teil-Komponenten bestehen, die gegebenenfalls auch räumlich verteilt sein können.

**Patentansprüche**

1. Computerimplementiertes Verfahren zur Ermittlung eines Performanzwerts einer MRT-Anlage, wobei das Verfahren umfasst:

   Empfangen einer Referenz-Häufigkeitsverteilung von Referenz-Depositionswerten untersuchter Referenz-Objekte,
   Empfangen zumindest eines Untersuchungs-Depositionswerts für eine MRT-Untersuchung zumindest eines Untersuchungs-Objekts mittels der MRT-Anlage,
   Ermitteln des Performanzwerts der MRT-Anlage anhand der Referenz-Häufigkeitsverteilung und des zumindest einen Depositionswerts.

2. Verfahren nach Anspruch 1, wobei jeder der Referenz-Depositionswerte und des zumindest eine Untersuchungs-Depositionswerts jeweils einen SAR-Wert und/oder einen B1-Wert und/oder einen Leistungswert, insbesondere einen Sendeleistungswert, beschreibt.

3. Verfahren nach einem der vorangehenden Ansprüche, wobei der zumindest ein Untersuchungs-Depositionswert anhand eines Kalibrierungsparameters ermittelt ist, der unabhängig von einem vorgegebenen MRT-Protokoll der MRT-Untersuchung ist.

4. Verfahren nach Anspruch 3, wobei der Kalibrierungsparameter einen Anlagensteuerwert zur Erzeugung eines Kalibrierungs-Hochfrequenzpulses mittels der MRT-Anlage beschreibt.

5. Verfahren nach einem der vorangehenden Ansprüche, wobei die Referenz-Häufigkeitsverteilung aus Referenz-Depositionswerten und/oder vorliegenden MRT-Untersuchungsdatensätzen mehrerer MRT-Referenz-Anlagen ermittelt ist.

6. Verfahren nach Anspruch 5, wobei die mehreren MRT-Referenz-Anlagen gleichen Typs sind.

7. Verfahren aus einem der vorangehenden Ansprüche, wobei das Verfahren ferner umfasst:

Empfangen zumindest eines Untersuchungsparameterwerts der MRT-Untersuchung, wobei die Referenz-Häufigkeitsverteilung aus vorliegenden MRT-Untersuchungsdatensätzen von untersuchten Referenz-Objekten ermittelt ist, die jeweils den zumindest einen Untersuchungsparameterwert aufweisen und/oder deren zumindest einer Untersuchungsparameterwert in einem Bereich liegt, der dem empfangenen zumindest einen Untersuchungsparameterwert zugeordnet ist.

8. Verfahren nach Anspruch 7, wobei der zumindest eine Untersuchungsparameterwert eine Masse des zumindest einen Untersuchungs-Objekts und/oder eine Lage eines Untersuchungsbereichs des Untersuchungs-Objekts und/oder eine Anregungsform zur Anregung des Untersuchungs-Objekts und/oder eine Zugehörigkeit des Untersuchungs-Objekts zu einer Personengruppe umfasst.

9. Verfahren nach einem der vorangehenden Ansprüche, wobei die Referenz-Häufigkeitsverteilung in Form einer Summenhäufigkeit oder einer Verteilungsfunktion empfangen wird und/oder aus einer Häufigkeit abgeleitet wird.

10. Verfahren nach einem der vorangehenden Ansprüche, wobei das Ermitteln des Performanzwerts ein Ermitteln zumindest eines Wahrscheinlichkeitswerts umfasst, der die Wahrscheinlichkeit beschreibt, dass ein Referenz-Objekt gemäß der Referenz-Häufigkeitsverteilung einen geringeren Depositionswert aufweist als der zumindest eine Untersuchungs-Depositionswert, oder der die Wahrscheinlichkeit beschreibt, dass ein Referenz-Objekt gemäß der Referenz-Häufigkeitsverteilung einen höheren Untersuchungs-Depositionswert aufweist als der zumindest eine Untersuchungs-Depositionswert.

11. Verfahren nach einem der vorangehenden Ansprüche,

wobei der zumindest eine Untersuchungs-Depositionswert mehrere Untersuchungs-Depositionswerte umfasst, wobei das Ermitteln des Performanzwerts umfasst:

Ermitteln jeweils eines Wahrscheinlichkeitswerts, insbesondere nach Anspruch 10, für jeden der mehreren Untersuchungs-Depositionswerte, Ermitteln eines Mittelwerts oder Medianwerts aus den mehreren Wahrscheinlichkeitswerten.

12. Verfahren nach Anspruch 11, wobei die mehreren Wahrscheinlichkeitswerte zum Ermitteln des Mittelwerts oder Medianwerts anhand von Untersuchungs-Depositionswerten ermittelt werden, die einem ausgewählten Zeitintervall ermittelt wurden.

13. Auswerteeinheit, die ausgebildet ist, ein Verfahren nach einem der vorangehenden Ansprüche auszuführen, mit einer Empfangseinheit zum Empfangen einer Referenz-Häufigkeitsverteilung von Referenz-Depositionswerten untersuchter Referenz-Objekte, und einer Empfangseinheit zum Empfangen zumindest eines Untersuchungs-Depositionswerts für eine MRT-Untersuchung zumindest eines Untersuchungs-Objekts mittels der MRT-Anlage und eine Ermittlungseinheit zum Ermitteln des Performanzwerts der MRT-Anlage anhand der Referenz-Häufigkeitsverteilung und des zumindest einen Untersuchungs-Depositionswerts.

14. System umfassend eine Auswerteeinheit nach Anspruch 13, einer Datenbank und mehrere MRT-Referenz-Anlagen, die der Datenbank Referenz-Depositionswerte zur Erzeugung mindestens einer Referenz-Häufigkeitsverteilung bereitstellen.

15. Computerprogrammprodukt, welches ein Programm umfasst und direkt in einen Speicher einer Auswerteeinheit ladbar ist, mit Programmmitteln, um ein Verfahren nach einem der Ansprüche 1 bis 12 auszuführen, wenn das Programm in der Auswerteeinheit ausgeführt wird.

FIG 1

EP 4 509 859 A1

# FIG 2

```
┌──────────────────────────────┐
│                              │────S10
└──────────────────────────────┘
               │
               ▼
┌──────────────────────────────┐
│                              │────S20
└──────────────────────────────┘
               │
               ▼
┌────────────────────────────────┐
│ ┌────────────────────────────┐ │
│ │                            │ │────S31
│ └────────────────────────────┘ │
│              │                 │────S30
│              ▼                 │
│ ┌────────────────────────────┐ │
│ │                            │ │
│ └────────────────────────────┘ │────S32
└────────────────────────────────┘
```

FIG 3

EP 4 509 859 A1

FIG 4

EP 4 509 859 A1

Europäisches Patentamt

European Patent Office

Office européen des brevets

# EUROPÄISCHER RECHERCHENBERICHT

Nummer der Anmeldung

EP 23 19 1605

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | QIAN DI ET AL: "An RF dosimeter for independent SAR measurement in MRI scanners", MEDICAL PHYSICS, AIP, MELVILLE, NY, US, Bd. 40, Nr. 12, 15. November 2013 (2013-11-15), XP012178851, ISSN: 0094-2405, DOI: 10.1118/1.4829527 | 1-9, 11-15 | INV. G01R33/54 G01R33/58 ADD. G01R33/28 |
| A | * Rechte Spalte auf Seite 122303-2 Abschnitte 2.A,2.D, 2.E, 2.F, 2.G, 3.A, 3.B und 4; Abbildungen 5-7 * ----- | 10 | |

RECHERCHIERTE SACHGEBIETE (IPC)

G01R
G16H

Der vorliegende Recherchenbericht wurde für alle Patentansprüche erstellt

| Recherchenort | Abschlußdatum der Recherche | Prüfer |
|---|---|---|
| München | 12. Januar 2024 | Lebar, Andrija |

EPO FORM 1503 03.82 (P04C03)